# EUROPÄISCHE PATENTSCHRIFT

(11) **EP 0 658 630 B1**
(45) Veröffentlichungstag und Bekanntmachung des Hinweises auf die Patenterteilung: **16.08.2001**
(21) Anmeldenummer: 94116541.7
(22) Anmeldetag: 20.10.1994
(51) Int. Cl.: C12Q 1/68

(54) **Oligonukleotide für den Nachweis von Enterobacteriaceae**
Oligonucleotides for the detection of enterobacteriaceae
Oligonucléotides pour la détection d'entérobactériacéae

(30) Priorität: 02.11.1993 DE 4337295
(43) Veröffentlichungstag der Anmeldung: 21.06.1995
(73) Patentinhaber: MERCK PATENT GmbH, 64293 Darmstadt (DE)
(72) Erfinder: Bubert, Dr. Andreas, D-97218 Gerbrunn (DE); Goebel, Dr. Prof. Werner, D-97218 Gerbrunn (DE); Goetz, Monika, D-97218 Gerbrunn (DE); Ludwig, Albrecht, D-97080 Würzburg (DE); Schubert, Dr. Peter, D-64285 Darmstadt (DE); Neumann, Dr. Siegfried, D-64342 Seeheim-Jugenheim (DE)

(56) Entgegenhaltungen:
- EP-A- 0 355 989
- EP-A- 0 383 509
- WO-A-94/25595
- LIBBY S ET AL: "A CYTOLYTIC TOXIN ENCODED BY SALMONELLA IS REQUIRED FOR VIRULENCE AND SURVIVAL WITHIN MACROPHAGES." 93RD GENERAL MEETING OF THE AMERICAN SOCIETY FOR MICROBIOLOGY, ATLANTA, GEORGIA, USA, MAY 16-20, 1993. , XP002045474 & : ABSTR GEN MEET AM SOC MICROBIOL, Bd. 93, Nr. 0, 1993, Seite 39
- RAHN ET AL.: "Amplification of an invA gene sequence of S. typhimurum by PCR as a specific method of detection of Salmonella" MOLECULAR AND CELLULAR PROBES, Bd. 6, 1992, LONDON GB, Seiten 271-279, XP002045475
- SPIERINGS ET AL.: "Characterization of the S. typhimurium phoE gene and development of Salmonella-specific probes" GENE, Bd. 122, 1992, AMSTERDAM NL, Seiten 45-52, XP002045476
- LIBBY ET AL.: "A cytolysin encoded by Salmonella is required for survival within macrophages" PROCEEDINGS OF THE NATIONAL ACADEMY OF SCIENCES OF USA, Bd. 91, Januar 1994, WASHINGTON US, Seiten 489-493, XP002045477

## Beschreibung

Die Erfindung betrifft Oligonukleotide, die als Nukleinsäuresonden oder als Primer geeignet sind für den Nachweis von Enterobacteriaceae, insbesondere von pathogenen Enterobacteriaceae, wie z.B. Salmonella sp..

Aus der Familie der Enterobacteriaceae sind die Vertreter Escherichia coli, Salmonella sp. und Shigella sp. die häufigsten Verursacher von Lebensmittelvergiftungen. Insbesondere durch Salmonellen verursachte Lebensmittelvergiftungen nehmen in jüngster Zeit erheblich zu; dabei stieg sowohl die Häufigkeit, wie auch die Schwere der Erkrankungen.

Voraussetzung für eine schnelle und gezielte Behandlung von Erkrankten und für die rasche Eindämmung des Infektionsherdes sind schnelle und spezifische Nachweisverfahren für den Nachweis von Salmonellen und anderen pathogenen Enterobacteriaceae. Gesetzliche Regelungen verlangen für den Nachweis von Salmonellen, daß die verwendeten Nachweismethoden für diese Bakterien unabhängig von Pathogenität und Virulenz sensitiv sein müssen. Für den Nachweis von Salmonellen sind Methoden üblich, die eine mehrstufige Kultur und eine serologische Typisierung umfassen. Es dauert mehrere Tage, bis die nach diesen Methoden erzielbaren Ergebnisse vorliegen. Immunologische Methoden sind zwar schneller, sie sind jedoch nur soweit anwendbar, wie sichergestellt werden kann, daß der verwendete Marker stets exprimiert wird; daraus ergeben sich zwangsweise Einschränkungen. Diese Einschränkungen gelten nicht für Nachweismethoden, die auf der Verwendung von Nukleinsäuresonden beruhen. Jedoch ist der Nachweis mit Nukleinsäuresonden im allgemeinen nicht so empfindlich, daß auf eine Vorkultur verzichtet werden kann. Aus diesem Grund ist das Kolonie-Hybridisierungsverfahren eine gebräuchliche Verfahrensvariante.

Nachweisverfahren, die auf Nukleinsäureamplifikationsreaktionen beruhen, z.B. die Polymerase-Kettenreaktion (polymerase chain reaction; PCR), verbessern die Nachweisgrenze, so daß auf die Vorkultur im allgemeinen verzichtet werden kann.

Verfahren zum Nachweis von Salmonellen basierend auf der Polymerase-Kettenreaktion sind aus der Literatur bekannt:
a) Spierungs, G. et al. (1992) Gene **122**, 45-52, benutzten Primer aus dem phoE Gen von S. typhimurium. Bei der Nachweisreaktion wurden jedoch nicht alle untersuchten Stämme der Gattung Salmonella erkannt.
b) Rahn, K. et al. (1992) Molecular and Cellular Probes **6**, 271-279, benutzten Primer aus dem invA Gen. Bei der Nachweisreaktion wurden jedoch nicht alle untersuchten Stämme der Gattung Salmonella erkannt; auch traten bei Kontrollversuchen mit anderen Enterobacteriaceae PCR-Produkte auf.

Aus EP-A-0 355 989 und EP-A-0 383 509 sind Verfahren für den Nachweis von Salmonellen mittels Nukleinsäuresonden bekannt. Nachgewiesen werden dabei Sequenzen aus dem araC-Gen beziehungsweise aus einem Gen, das eine bestimmte Gruppe von Fimbrien codiert.

Weil der Nachweis von Salmonellen eine extrem hohe diagnostische Sensitivität erfordert, besteht also die Aufgabe weiterhin, verbesserte Nachweismethoden, basierend auf der Nukleinsäuresondentechnik und/oder auf Nukleinsäureamplifikationsreaktionen, bereitzustellen.

Es wurde gefunden, daß eine Gruppe von Genen, die bei pathogenen E. coli Stämmen für die Expression von hämolysierenden Toxinen verantwortlich sind, auch in anderen Enterobacteriaceae vorkommen. Dieser Befund war überraschend, da viele der untersuchten Stämme keine hämolysierenden Toxine bilden, aber dennoch diese Gene aufweisen.

Bei Salmonellen, die hämolysierende Toxine bilden, wurden zwei dieser Gene erstmals entdeckt und slyA und slyC genannt. Es wurde gefunden, daß Teilsequenzen ausgewählt aus dem Genbereich slyA und slyC für spezifische Nachweisverfahren geeignet sind. Diese erfindungsgemäßen Nachweisverfahren können auf der Nukleinsäuresondentechnik und/oder auf Nukleinsäureamplifikationsreaktionen beruhen; sie erlauben den Nachweis von Bakterien der Gattung Salmonella und/oder anderer Enterobacteriaceae mit hoher Spezifität.

Unter der Definition "Salmolysin-DNA-Region" werden erfindungsgemäß die DNA-Bereiche verstanden, die die für die Funktion von Salmolysin notwendigen kodierenden Abschnitte (SlyA und SlyC) einschließlich deren flankierenden nicht-kodierenden Bereiche bei der Gattung Salmonella beinhalten. Unter der "Salmolysin-ähnlichen DNA-Region" werden die DNA-Bereiche verstanden, die diese Abschnitte bei Bakterien der Gattung Salmonella und bei anderen Enterobacteriaceae beinhalten.

Gegenstand der Erfindung sind Oligonukleotide, ausgewählt aus der Salmolysin-ähnlichen DNA-Region, für den Nachweis von Enterobacteriaceae, dadurch gekennzeichnet, daß sie eine Teilsequenz nach einer der Formeln la bis Ih oder eine zugehörige Komplementärsequenz enthalten, wobei vor und/oder hinter der jeweiligen Teilsequenz bis zu 20 weitere Nukleotidbausteine gebunden vorliegen können:

TAT ATT GCG TTA GAT T Ia

CTG AAG CTA CAG GT Ib

AGC AAG CTA ATT ATA Ic

ACG GTT GGT GCG CAT T Id

ATA CGT GTG GCC ATG | Ie

GTG TGG TCA GTA ACC | If

CTG GCC AGC ACG AC | Ig

TCA TCA CCG CCC TG Ih

Besonders bevorzugt sind dabei Oligonukleotide mit einer Sequenz nach einer der Formeln IIIa bis IIIh oder mit einer zugehörigen Komplementärsequenz:

GAA TAT ATT GCG TTA GAT TAA T IIIa

AAA CTG AAG CTA CAG GTG CC IIIb

CTT AGC AAG CTA ATT ATA AGG IIIc

GGC ACG GTT GGT GCG CAT TTG G IIId

GGC ATA CGT GTG GCC ATG TGA IIIe

GGC GTG TGG TCA GTA ACC TGA IIIf

TTG CTG GCC AGC ACG ACA CG IIIg

GAA TCA TCA CCG CCC TGA AT IIIh

Gegenstand der Erfindung ist die Verwendung eines Oligonukleotids nach einer der Formeln lla-h und/oder IIIa-h als Nukleinsäuresonde oder als Primer für den Nachweis von Enterobacteriaceae, insbesondere von Bakterien der Gattung Salmonella.

Gegenstand der Erfindung sind Verfahren zum Nachweis von Enterobacteriaceae, inbesondere von Bakterien der Gattung Salmonella, dadurch gekennzeichnet, daß ein Oligonukleotid nach einer der Formeln lla-h und/ oder IIIa-h als Nukleinsäuresonde oder als Primer verwendet wird.

Gegenstand der Erfindung sind Testzusammenstellungen zum Nachweis von Enterobacteriaceae, insbesondere von Bakterien der Gattung Salmonella, dadurch gekennzeichnet, daß darin ein Oligonukleotid nach einer der Formeln lla-h und/oder IIIa-h als Nukleinsäuresonde oder als Primer enthalten ist.

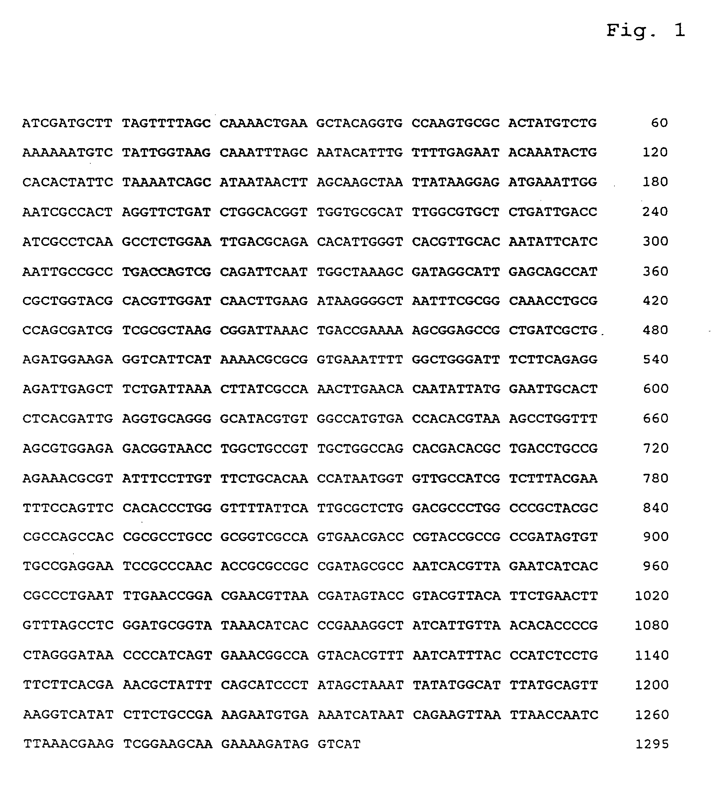

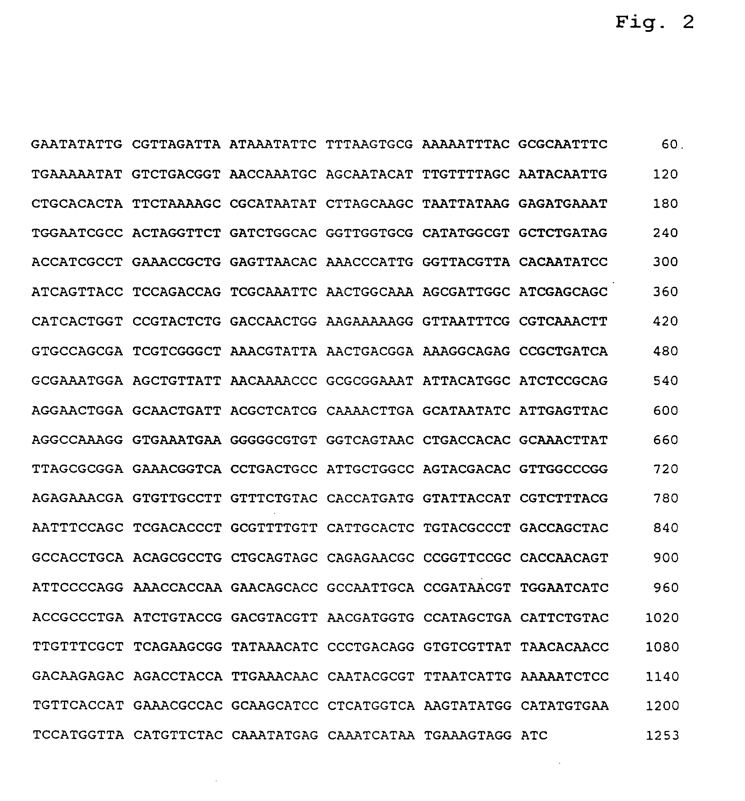

Die Abbildung 1 stellt die Sequenz der DNA-Region dar, die in Bakterien der Gattung Salmonella für Salmolysin-Genprodukte kodiert; Abbildung 2 stellt die Sequenz der entsprechenden DNA- Region aus E. coli dar. Der Sequenzbereich von Position 1 bis 972 von Shigella flexneri ist homolog zu dem von E. coli.

Die Erfindung wird im folgenden näher beschrieben. Dabei wird in der Regel auf Einzelheiten von biochemischen, immunologischen und molekularbiologischen Verfahren, die dem Fachmann bekannt sind, und deren Einzelheiten in der Literatur beschrieben sind, nicht näher eingegangen. Bei diesen Verfahren kann man auch von an sich bekannten, hier nicht näher beschriebenen Varianten Gebrauch machen.

Dem Fachmann ist bekannt, daß häufig der Austausch von einer oder weniger Basen in einer Nukleotidsequenz deren biologische Eigenschaften nicht verändert. So ist es bekannt, daß zwei Nukleinsäureabschnitte auch hybridisieren können, wenn einzelne Basen nicht genau der Komplementärstruktur entsprechen.

Basierend auf der jeweiligen Sequenz können die erfindungsgemäßen Oligonukleotide nach dem Fachmann bekannten Verfahren, beispielsweise der Phosphotriester- oder der Phosphoamidit-Methode, synthetisiert werden. Bevorzugt wird die Phosphoamidit-Methode benutzt, insbesondere unter Verwendung von mechanisierten Synthetizem. Die Methode ist in Tetrahedron Lett. (1981) **22** : 1859-1862 beschrieben. Weitere Einzelheiten derartiger Syntheseverfahren sind beispielsweise in Winnacker, E.L. (1985) Gene und Klone, Seite 44-61 (VCH-Verlagsgesellschaft mbH, Weinheim), beschrieben.

Die erfindungsgemäßen Oligonukleotide nach den Formeln lla - IIh und llla - IIIh sind als Nukleinsäuresonden und somit zum spezifischen Nachweis von Enterobacteriaceae, insbesonders von Bakterien der Gattung Salmonella geeignet. Ihre Sequenzen sind in üblicher Weise vom 5'-Ende zum 3'-Ende geschrieben dargestellt. Da Nukleinsäuresonden mit einer Zielsequenz hybridisieren, und da es zu jeder Zielsequenz stets eine komplementäre Sequenz auf dem Gegenstrang gibt, sind auch Oligonukleotide, die eine Sequenz komplementär zu einer der Formeln II a-h oder III a-h aufweisen, in gleicher Weise erfindungsgemäß geeignet.

Nukleinsäuresonden werden üblicherweise mit Markierungsmitteln (tracer) verbunden, um den analytischen Nachweis zu erlauben. Beispielsweise können die Sonden selbst radioaktiv mit P-32 oder H-3 markiert sein. Die Verfahren zur Isotopenmarkierung und weitere zur Markierung geeignete Radioisotope sind dem Fachmann bekannt. Neben diesen radioaktiven Markierungsmitteln sind weitere nicht-isotopische Markierungsmittel dem Fachmann geläufig. Diese werden bei analytischen Aufgaben häufig bevorzugt. Diese Markierungen werden an die Sonde, beispielsweise mit Brückenmolekülen, gebunden. Geeignete nicht-isotopische Markierungsmittel sind beispielsweise fluoreszierende Stoffe, wie z.B. Fluoreszein, oder auch Enzyme, wie z.B. Peroxidase oder Alkalische Phosphatase. Die Auswahl von geeigneten fluoreszierenden Stoffen oder von Enzymen und die notwendigen Nachweismethoden sind dem Fachmann bekannt. In vielen Fällen ist es sinnvoll, die Markierungsmittel nicht direkt mit der Sonde, sondern indirekt mittels zusätzlicher stark bindender Liganden zu verbinden. Für diesen Zweck haben sich insbesondere die Kombinationen Biotin/Avidin oder Biotin/Streptavidin bewährt. Die Auswahl derartiger Liganden und die notwendigen Bindungsverfahren sind dem Fachmann bekannt. Erfindungsgemäß wird entsprechend dieser Beispiele unter dem Begriff Markierungsmittel sowohl die bei direkten Markierungsverfahren benutzten Markierungsmittel als auch die bei indirekten Markierungsverfahren benutzten Kombinationen unter Einschluß der bindenden Liganden verstanden. Unter dem Begriff "Nukleinsäuresonde" wird erfindungsgemäß sowohl die als Sonde benutzte Nukleinsäure als auch die Verbindung aus Nukleinsäure und Markierungsmittel verstanden.

Die erfindungsgemäßen Oligonukleotide nach den Formeln lla - IIh und llla - IIIh sind auch als Primer für Nukleinsäureamplifikationsmethoden und somit zum spezifischen Nachweis von Enterobacteriaceae, insbesonders von Bakterien der Gattung Salmonella geeignet, eine Vorkultur ist bei diesen Verfahren im allgemeinen nicht notwendig. In Abhängigkeit von den Erfordernissen des jeweils verwendeten Amplifikationssystems werden entweder Desoxyribonukleotide oder auch Ribonukleotide mit den erfindungsgemäßen Sequenzen eingesetzt. Im letzteren Fall sind die Thymidinbausteine jeweils durch Uridinbausteine ersetzt. Da üblicherweise je ein Primer mit jeweils einem der DNA-Stränge reagieren soll, wird einer der Primer in der komplementären Sequenz eingesetzt. Die komplementäre Sequenz ergibt sich in bekannter Weise nach den Regeln der Basenpaarung.

Die erfindungsgemäßen Primer sind geeignet für DNS-Amplifikation, beispielsweise mit Hilfe der Polymerase Chain Reaction (PCR). Dazu wird die DNS durch Erwärmen zunächst in die Einzelstränge zerlegt. Es werden zwei Primer verwendet, die jeweils mit dem homologen DNS-Abschnitt auf jeweils einem DNS-Strang hybridisieren. Der Genomabschnitt, der zwischen diesen beiden Primern liegt, wird vermehrt. Die an die DNS angelagerten Primer stellen die Startpunkte für die Amplifikation dar. Eine Polymerase, bevorzugt taq-DNA-Polymerase, ergänzt anschließend in Gegenwart der vier Nukleotidtriphosphate den zweiten Strang entsprechend der Sequenz der ursprünglichen DNA. Anschließend werden die entstandenen Doppelstränge wieder durch Erwärmen in die Einzelstränge zerlegt. Dieser Amplifikationszyklus kann mehrfach wiederholt werden. Nach einer ausreichenden Anzahl von Amplifikationszyklen kann die amplifizierte Nukleinsäure mittels bekannter Methoden nachgewiesen werden. Dazu kann die DNS mittels Elektrophorese aufgetrennt, anschließend mit Ethidiumbromid angefärbt und schließlich durch Fluoreszenz mittels UV-Anregung nachgewiesen werden. Möglich ist auch der Nachweis mittels DNS- Hybridisierung. Die Einzelheiten geeigneter Amplifikations- und Nachweismethoden sind in Übersichtsartikeln, z.B. Innis et al. (eds.) PCR Protocols (Academic Press, Inc., Harcourt Brace Jovanovich, Publishers) beschrieben. Ebenso sind andere Nukleinsäureamplifikationsverfahren, bei denen die erfindungsgemäßen Primer benutzt werden können, aus der Literatur bekannt. Zu diesen gehört die Ligase-Ketten-Reaktion, beschrieben von Bond, S. et al. (1990), Seite 425-434 bei Raven Press (New York, NY/USA).

Die Auswahl eines der beiden Primer ist stets besonders kritisch, während der zweite Primer eher variiert werden kann, ohne die Spezifität der Nachweisreaktion nennenswert zu verändern. Folglich kann nach der Lehre der vorliegenden Erfindung für diesen zweiten Primer durchaus auch eine Sequenz gewählt werden, die nicht einer der Formeln IIa - llh oder IIIa - IIIh entspricht.

Erfindungsgemäß wird zumindestens einer der Primer aus den Formeln IIa - IIh oder bevorzugt aus den Formeln llla - IIIh ausgewählt. Der zweite Primer beeinflußt, wie bereits erläutert, die Spezifität der Amplifikationsreaktion wesentlich weniger als der erste Primer. Bevorzugt werden jedoch Kombinationen, bei denen beide Primer aus den Formeln IIa - IIh oder IIIa - IIIh ausgewählt werden.

In den bereits genannten Publikationen von Spierungs, G. et al. (1992) und Rahn, K. et al. (1992) finden sich nähere Hinweise zu Einzelheiten des PCR-Verfahrens.

Es ist auch möglich, die Amplifikationsprodukte durch Nukleinsäurehybridisierung nachzuweisen. Dazu werden dem Reaktionsansatz nach der Amplifikation Nukleinsäuresonden, die mit dem amplifizierten Abschnitt hybridisieren, zugesetzt.

Einzelheiten der Herstellung der erfindungsgemäßen Oligonukleotide, sowie ihrer Verwendung sind aus den folgenden Beispielen ersichtlich. Weitere methodische Einzelheiten entnimmt der Fachmann der zitierten Literatur. Die Beispiele sollen den Gegenstand der Erfindung erläutern und stellen keine Einschränkung der Erfindung dar.

### Beispiele:

### Beispiel 1: Herstellung des Oligonukleotids nach Formel IIIb

Das Oligonukleotid nach Formel IIIb wird mit dem DNA-Syntheziser 380A von Applied Biosystems nach der Phosphoamidit-methode hergestellt. Die Grundzüge der Methode sind in Tetrahedron Lett. (1981) **22**: 1859-1862, beschrieben. Weitere Einzelheiten finden sich in der Dokumentation des Geräteherstellers.

Entsprechend werden die Oligonukleotide nach Formel IIIa, IIIc und IIId hergestellt. Die Oligonukleotide nach Formel IIIf, IIIg und IIIh werden in der jeweiligen Komplementärsequenz hergestellt. Das Oligonukleotid nach Formel IIIe wird sowohl in der angegebenen Sequenz als auch in der zugehörigen Komplementärsequenz hergestellt.

Die solchermaßen erhaltenen Oligonukleotide können entweder als DNA-Sonde oder als Primer für die PCR-Reaktion Verwendung finden.

### Beispiel 2: Markierung einer DNA-Sonde mit P-32

Ein nach Beispiel 1 erhaltenes Oligonukleotid wird radioaktiv markiert, wie in Abschnitt 4.8 von "Current Protocols in Molecular Biology" (1988); Verlag Wiley and Sons, New York, beschrieben:
1 µl Oligonukleotidlösung, enthaltend 100 ng Oligonukleotid, werden mit 20 µl einer Lösung von gamma-³²P-ATP (200 µCi), mit 2,5 µl eines zehnfach konzentrierten Kinasepuffers (0,7 M Tris-HCI; pH 7,5; 0,1 M MgCl₂; 50 mM Dithiothreitol, 1 mM Spermidin-HCI; 1 mM EDTA) und mit 4 Einheiten T4-Polynukleotidkinase (Fa. Pharmacia) versetzt und 30 Minuten bei 37 °C inkubiert. Anschließend wird das Enzym durch Erwärmen (65 °C; 5 Minuten) inaktiviert. Das markierte Oligonukleotid wird durch Zugabe von 25 µl 4 M Ammoniumacetatlösung und 250 µl absolutem Ethanol gefällt und zentrifugiert (15 Minuten; 12000 UpM). Der Niederschlag wird dreimal in jeweils 25 µl Wasser suspendiert und wieder zentrifugiert.

### Beispiel 3: Hybridisierung mit einer radioaktiv markierten DNA-Sonde (Kolonie-Hybridisierungsverfahren)

Das Kolonie-Hybridisierungsverfahren wird nach der Vorschrift von Datta et al. (1988) Appl. and Environmental Microbiol. **54**: 2933-2937, ausgeführt:
a) Vorbehandlung:
   Bakterien aus der Untersuchungsprobe werden auf einer Petrischale vorkultiviert (Brain Heart Infusion; 18 Stunden; 37 °C). Anschließend wird ein Membranfilter (Hybond-C; Fa. Amersham) auf die Agaroberfläche mit den vorkultivierten Bakterien gelegt und leicht angedrückt. Der Filter wird, mit den Bakterien nach oben, auf ein Filterpapier (Whatman 3M) gelegt, das sich in einer mit 3 ml NaCl-Lösung (0,85 %; w/w) gefüllten Plastikpetrischale befindet, und wird 30 Sekunden bei 700 W in einem Mikrowellenofen behandelt. Anschließend wird der Filter mit 3 ml Lysis-Lösung (0,5 M NaOH, 1,5 M NaCI) 5 Minuten bei Raumtemperatur behandelt und dann kurz auf ein mit Neutralisierungslösung (1 M Tris-HCI; pH 7,0; 2 M NaCI) getränktes Filterpapier gelegt, anschließend 5 Minuten in Neutralisierungslösung inkubiert und an der Luft getrocknet.
b) Vorhybridisierung:
   Die Vorhybridisierung der DNA, die auf den Membranfilter aufgezogen ist, erfolgt mit 100 µg/ml denaturierter DNA aus Heringssperma für vier Stunden bei 45 °C in Hybridisierungspuffer mit folgender Zusammensetzung: 0,6 M Tris-HCl pH 8,0, 6 M NaCI und 60 mM EDTA (sechsfach konzentrierte STE-Lösung), 0,1 % Ficoll^{R}, 0,1 % Polyvinylpyrrolidon und 0,1 % Rinderserumalbumin (fünffach konzentrierte Lösung nach Denhardt), 0,1 % Natriumdodecylsulfat, sowie 100 µg/ml denaturierte DNA aus Heringssperma.
c) Hybridisierung:
   Für die Hybridisierung wird die radioaktiv markierte Oligonukleotidsonde nach Formel IIIe, hergestellt nach Beispiel 1, zusammen mit dem Hybridisierungspuffer zu dem Membranfilter mit der aufgezogenenen DNA zugegeben. Die Hybridisierung erfolgt über 18 Stunden bei 45 °C. Anschließend wird der Filter gewaschen: dreimal für je 5 Minuten bei Raumtemperatur mit 0,1 % Natriumdodecylsulfat in doppelt konzentrierter SSC-Lösung (0,3 M NaCl in 30 mM Trinatriumcitrat; pH 7,0), dreimal für jeweils 20 Minuten bei 45 °C in 0,1 % Natriumdodecylsulfat in SSC-Lösung und einmal in SSC-Lösung bei Raumtemperatur.
   Anschließend wird das Filter auf einen Röntgenfilm (Fuji RX Medical X-Ray film, Fa. Fuji Photo Film Co.) aufgelegt und der Film über Nacht exponiert. Nach der Entwicklung zeigt die Schwarzfärbung auf Kolonien von Salmonellen hin.

### Beispiel 4: Durchführung der PCR-Reaktion zum genusspezifischen Nachweis von Salmonella

Eine bakterienhaltige Probe mit ca. 1 µg DNA wird in 50 µl Puffer (10 mM Tris-HCI pH 8,5; 1,5 mM MgCl₂ und 50 mM KCI) suspendiert und 5 Minuten auf 110 °C erhitzt. Anschließend werden Primer nach Formel IIIb und IIIh (siehe Beispiel 1; je 0,4 µg), sowie 2,5 U Taq-Polymerase (Fa. Pharmacia), gelöst in Reaktionspuffer (10 mM Tris-HCI pH 8,5; 1,5 mM MgCl₂ und 50 mM KCI), sowie jeweils 200 µM dGTP, dATP, dTTP und dCTP zugefügt (gesamtes Reaktionsvolumen 100 µl). Der erste Denaturierungsschritt dauert 3 Minuten bei 94 °C. Anschließend wird für 30 Sekunden auf 55 °C (Bindungsphase) und für eine Minute auf 72 °C (Elongationsphase) temperiert. Die folgenden Denaturierungsschritte (bei 94 °C) dauern 45 Sekunden. Nach 30 Reaktionszyklen wird ein abschließender Elongationsschritt (bei 72 °C) mit 5 Minuten Dauer ausgeführt.

Die PCR-Produkte werden in einem Polyacrylamid-Gel (6 %) in einem Laufpuffer Tris-Borat (je 50 mM) mit EDTA (2,5 mM) aufgetrennt. Anschließend werden die aufgetrennte PCR-Produkte durch Anfärbung mit Ethidiumbromid (0,1 mg/ml in Wasser) angefärbt und Bestrahlung mit UV-Licht (260 nm) sichtbar gemacht.

Nur wenn DNA oder Zellen von Salmonellen in der Probe vorhanden sind, werden PCR-Produkte beobachtet (siehe Spalte a) in Tabelle 1).

### Beispiel 5: Durchführung der PCR-Reaktion zum genusspezifischen Nachweis von Salmonella

Das in Beispiel 4 beschriebene Verfahren wird unter Verwendung der Primer nach Formel IIIe und IIIh (siehe Beispiel 1) anstelle der Primer nach Formel IIIb und IIIh wiederholt. Auch in diesem Fall werden nur PCR-Produkte beobachtet, wenn DNA oder Zellen von Salmonella in der Probe vorhanden sind (siehe Spalte b) in Tabelle 1).

### Beispiel 6: Durchführung der PCR-Reaktion zum genusspezifischen Nachweis von Salmonella

Das in Beispiel 4 beschriebene Verfahren wird unter Verwendung der Primer nach Formel IIIb und IIIe (komplementäre Sequenz) (siehe Beispiel 1) anstelle der Primer nach Formel IIIb und IIIh wiederholt. Auch in diesem Fall werden nur PCR-Produkte beobachtet, wenn DNA oder Zellen von Salmonella in der Probe vorhanden sind.

### Beispiel 7: Durchführung der PCR-Reaktion zum gruppenspezifischen Nachweis der Enterobacteriaceae Salmonella, Citrobacter, E. coli und Shigella

Eine bakterienhaltige Probe mit ca. 1 µg DNA wird in 50 µl Puffer (10 mM Tris-HCI pH 8,5; 1,5 mM MgCl₂ und 50 mM KCI) suspendiert und 5 Minuten auf 110 °C erhitzt.

Anschließend werden Primer nach Formel IIIc und IIIh (siehe Beispiel 1; je 0,4 µg), sowie 2,5 U TaqPolymerase (Fa. Pharmacia), gelöst in Reaktionspuffer (10 mM Tris-HCI pH 8,5; 1,5 mM MgCl₂ und 50 mM KCI), sowie jeweils 200 µM dGTP, dATP, dTTP und dCTP zugefügt (gesamtes Reaktionsvolumen 100 µl). Der erste Denaturierungsschritt dauert 3 Minuten bei 94 °C. Anschließend wird für 30 Sekunden auf 55 °C (Bindungsphase) und für eine Minute auf 72 °C (Elongationsphase) temperiert. Die folgenden Denaturierungsschritte (bei 94 °C) dauern 45 Sekunden. Nach 30 Reaktionszyklen wird ein abschließender Elongationsschritt (bei 72 °C) mit 5 Minuten Dauer ausgeführt.

Die PCR-Produkte werden in einem Polyacrylamid-Gel (6 %) in einem Laufpuffer Tris-Borat (je 50 mM) mit EDTA (2,5 mM) aufgetrennt. Anschließend werden die aufgetrennte PCR-Produkte durch Anfärbung mit Ethidiumbromid (0,1 mg/ml in Wasser) angefärbt und Bestrahlung mit UV-Licht (260 nm) sichtbar gemacht.

Nur wenn DNA oder Zellen der Enterobacteriaceae Salmonella, Citrobacter, E. coli und Shigella in der Probe vorhanden sind, werden PCR-Produkte beobachtet (siehe Spalte c) in Tabelle 1).

### Beispiel 8: Durchführung der PCR-Reaktion zum gruppenspezifischen Nachweis der Enterobacteriaceae Salmonella, Citrobacter, E. coli und Shigella

Das in Beispiel 7 beschriebene Verfahren wird unter Verwendung der Primer nach Formel IIId und IIIh (siehe Beispiel 1) anstelle der Primer nach Formel IIIc und IIIh wiederholt. Auch in diesem Fall werden nur PCR-Produkte beobachtet, wenn DNA oder Zellen der Enterobacteriaceae Salmonella, Citrobacter, E. coli und Shigella in der Probe vorhanden sind (siehe Spalte d) in Tabelle 1).

**Tabelle 1:**

| | **a)** | **b)** | **c)** | **d)** |
|---|---|---|---|---|
| Primer 1 | IIIb | IIIe | IIIc | IIId |
| Primer 2 (Komplementärsequenz) | IIIh | IIIh | IIIh | IIIh |
| Bakterienstamm | | | | |
| Salmonella | | | | |
| arizonae | + | + | + | + |
| choleraesuis | + | + | + | + |
| enteritidis | + | + | + | + |
| infantis | + | + | + | + |
| paratyphi A | + | + | + | + |
| paratyphi B | + | + | + | + |
| typhi | + | + | + | + |
| typhimurium | + | + | + | + |
| bovis morbificans | + | + | + | + |
| brandenburg | + | + | + | + |
| bredeney | + | + | + | + |
| derby | + | + | + | + |
| heidelberg | + | + | + | + |
| london | + | + | + | + |
| montevideo | + | + | + | + |
| saintpaul | + | + | + | + |
| senftenberg | + | + | + | + |
| thomson | + | + | + | + |
| eimsbüttel | + | + | + | + |
| Citrobacter diversus | - | - | + | + |
| Escherichia coli | - | - | + | + |
| Shigella | | | | |
| flexneri | - | - | + | + |
| sonnei | - | - | + | + |
| Yersinia | - | - | - | - |
| Edwardsiella | - | - | - | - |
| Enterobacter aerogenes | - | - | - | - |
| Haemophilus influenzae | - | - | - | - |
| Hafnia alvei | - | - | - | - |
| Klebsiella pneumoniae | - | - | - | - |
| Neisseria meningitidis | - | - | - | - |
| Serratia | - | - | - | - |
| Pasteurella multocida | - | - | - | - |
| Proteus | - | - | - | - |
| mirabilis | - | - | - | - |
| vulgaris | - | - | - | - |
| Pseudomonas aeroginosa | - | - | - | - |
| Vibrio parahaemolyticus | - | - | - | - |

## Patentansprüche

1. Oligonukleotid, ausgewählt aus der Salmolysin-ähnlichen DNA-Region, für den Nachweis von Enterobacteriaceae, gekennzeichnet durch eine Sequenz nach einer der Formeln lla bis IIh und/oder einer zugehörigen Komplementärsequenz, worin
X¹ und X² jeweils unabhängig voneinander Wasserstoff oder ein bis 20 weitere Nukleotide
bedeuten:

X¹ TAT ATT GCG TTA GAT T X²          IIa

X¹ CTG AAG CTA CAG GT X² IIb

X¹ AGC AAG CTA ATT ATA X²          IIc

X¹ ACG GTT GGT GCG CAT T X²          IId

X¹ ATA CGT GTG GCC ATG X²    Ile

X¹ GTG TGG TCA GTA ACC X² IIf

X¹ CTG GCC AGC ACG AC X²     IIg

X¹ TCA TCA CCG CCC TG X² IIh

2. Oligonukleotid, gekennzeichnet durch eine Sequenz nach einer der Formeln IIIa bis IIIh, oder durch eine entsprechende Komplementärsequenz:

GAA TAT ATT GCG TTA GAT TAA T IIIa

AAA CTG AAG CTA CAG GTG CC IIIb

CTT AGC AAG CTA ATT ATA AGG IIIc

GGC ACG GTT GGT GCG CAT TTG G IIId

GGC ATA CGT GTG GCC ATG TGA IIIe

GGC GTG TGG TCA GTA ACC TGA IIIf

TTG CTG GCC AGC ACG ACA CG IIIg

GAA TCA TCA CCG CCC TGA AT IIIh

3. Verwendung eines Oligonukleotids nach einem der Ansprüche 1-2 als Nukleinsäuresonde oder als Primer für den Nachweis von Enterobacteriaceae, insbesondere von Bakterien der Gattung Salmonella.

4. Verwendung nach Anspruch 3, dadurch gekennzeichnet, daß der Nachweis mittels einer Nukleinsäuresonde erfolgt.

5. Verwendung nach Anspruch 3, dadurch gekennzeichnet, daß der Nachweis mittels Nukleinsäureamplifiktation erfolgt, wobei als Primer ein Oligonukleotid nach einem der Ansprüche 1-2 benutzt wird.

6. Verfahren zum Nachweis von Enterobacteriaceae, insbesondere von Bakterien der Gattung Salmonella, dadurch gekennzeichnet, daß ein Oligonukleotid nach einem der Ansprüche 1-2 als Nukleinsäuresonde oder als Primer verwendet wird.

7. Verfahren nach Anspruch 6, dadurch gekennzeichnet, daß der Nachweis mittels einer Nukleinsäuresonde erfolgt.

8. Verfahren nach Anspruch 6, dadurch gekennzeichnet, daß der Nachweis mittels Nukleinsäureamplifikation erfolgt, wobei als Primer ein Oligonukleotid nach einem der Ansprüche 1-2 benutzt wird.

9. Testzusammenstellung zum Nachweis von Enterobacteriaceae, insbesondere von Bakterien der Gattung Salmonella, dadurch gekennzeichnet, daß darin ein Oligonukleotid nach einem der Ansprüche 1-2 als Nukleinsäuresonde oder als Primer enthalten ist.

10. Testzusammenstellung nach Anspruch 9, dadurch gekennzeichnet, daß der Nachweis mittels einer Nukleinsäuresonde erfolgt.

11. Testzusammenstellung nach Anspruch 9, dadurch gekennzeichnet, daß der Nachweis mittels Nukleinsäureamplifikation erfolgt, wobei als Primer ein Oligonukleotid nach einem der Ansprüche 1-2 benutzt wird.

## Claims

1. Oligonucleotide, selected from the salmolysinlike DNA region, for the detection of Enterobacteriaceae, characterized by a sequence according to one of the formulae IIa to IIh and/or an affiliated complementary sequence, in which
X¹ and X², in each case independently of each other, are hydrogen or one to 20 additional nucleotides:

X^1 TAT ATT GCG TTA GAT T X^2 IIa

X¹ CTG AAG CTA CAG GT X²     IIb

X1 AGC AAG CTA ATT ATA X2 IIc

X¹ ACG GTT GGT GCG CAT T X²     IId

X^1 ATA CGT GTG GCC ATG X^2 IIe

X¹ GTG TGG TCA GTA ACC X²     IIf

X^1 CTG GCC AGC ACG AC X^2 IIg

X^1 TCA TCA CCG CCC TG X^2 IIh

2. Oligonucleotide, characterized by a sequence according to one of the formulae IIIa to IIIh, or by a corresponding complementary sequence:

GAA TAT ATT GCG TTA GAT TAA T  IIIa

AAA CTG AAG CTA CAG GTG CC IIIb

CTT AGC AAG CTA ATT ATA AGG IIIc

GGC ACG GTT GGT GCG CAT TTG G  IIId

GGC ATA CGT GTG GCC ATG TGA    IIIe

GGC GTG TGG TCA GTA ACC TGA    IIIf

TTG CTG GCC AGC ACG ACA CG IIIg

GAA TCA TCA CCG CCC TGA AT     IIIh

3. Use of an oligonucleotide according to one of Claims 1-2 as a nucleic acid probe or as a primer for the detection of Enterobacteriaceae, in particular of bacteria of the genus Salmonella.

4. Use according to Claim 3, characterized in that the detection is effected using a nucleic acid probe.

5. Use according to Claim 3, characterized in that the detection is effected by means of nucleic acid amplification, an oligonucleotide according to one of Claims 1-2 being employed as a primer.

6. Method for the detection of Enterobacteriaceae, in particular of bacteria of the genus Salmonella, characterized in that an oligonucleotide according to one of Claims 1-2 is used as a nucleic acid probe or as a primer.

7. Method according to Claim 6, characterized in that the detection is effected using a nucleic acid probe.

8. Method according to Claim 6, characterized in that the detection is effected by means of nucleic acid amplification, an oligonucleotide according to one of Claims 1-2 being employed as a primer.

9. Test compilation for the detection of Enterobacteriaceae, in particular of bacteria of the genus Salmonella, characterized in that the compilation contains an oligonucleotide according to one of Claims 1-2 as a nucleic acid probe or as a primer.

10. Test compilation according to Claim 9, characterized in that the detection is effected using a nucleic acid probe.

11. Test compilation according to Claim 9, characterized in that the detection is effected by means of nucleic acid amplification, an oligonucleotide according to one of Claims 1-2 being employed as a primer.

## Revendications

1. Oligonucléotide, choisi dans la région d'ADN de type Salmolysine, pour la recherche d'entérobactériacées, caractérisé par une séquence selon l'une des formules IIa à IIh et/ou une séquence complémentaire en faisant partie, dans lequel
X¹ et X² chacun indépendamment l'un de l'autre représentent l'hydrogène ou 1 à 20 autres nucléotides:

X¹ TAT ATT GCG TTA GAT T X²     IIa

X¹ CTG AAG CTA CAG GT X² IIb

X¹ AGC AAG CTA ATT ATA X²     IIc

X¹ ACG GTT GGT GCG CAT T X²     IId

X¹ ATA CGT GTG GCC ATG X²          IIe

X¹ GTG TGG TCA GTA ACC X²     IIf

X¹ CTG GCC AGC ACG AC X²          IIg

X¹ TCA TCA CCG CCC TG X²          IIh

2. Oligonucléotide , caractérisé par une séquence selon l'une des formules IIIa à IIIh, ou par une séquence complémentaire correspondante:

GAA TAT ATT GCG TTA GAT TAA T IIIa

AAA CTG AAG CTA CAG GTG CC IIIb

CTT AGC AAG CTA ATT ATA AGG IIIc

GGC ACG GTT GGT GCG CAT TTG G IIId

GGC ATA CGT GTG GCC ATG TGA IIIe

GGC GTG TGG TCA GTA ACC TGA IIIf

TTG CTG GCC AGC ACG ACA CG IIIg

GAA TCA TCA CCG CCC TGA AT IIIh

3. Utilisation d'un nucléotide selon l'une des revendications 1-2 comme sonde nucléique ou comme amorce pour la recherche des entérobactériacées, en particulier des bactéries du genre Salmonella.

4. Utilisation selon la revendication 3, caractérisée en ce que la recherche est effectuée avec une sonde d'acide nucléique.

5. Utilisation selon la revendication 3, caractérisée en ce que la recherche est effectuée par amplification d'acide nucléique, dans laquelle on utilise comme amorce un oligonucléotide selon l'une des revendications 1-2.

6. Procédé de recherche des entérobactériacées, en particulier des bactéries du genre Salmonella, caractérisé en ce que, un oligonucléotide selon l'une des revendications 1-2 est utilisé comme sonde d'acide nucléique ou comme amorce.

7. Procédé selon la revendication 6, caractérisé en ce que l'on effectue la recherche avec une sonde d'acide nucléique.

8. Procédé selon la revendication 6, caractérisé en ce que l'on effectue la recherche avec une amplification d'acide nucléique, dans laquelle on utilise comme amorce un oligonucléotide selon l'une des revendications 1-2.

9. Trousse d'essai pour la recherche des entérobactériacées, en particulier des bactéries du genre Samonella, caractérisée en ce qu'elle contient un oligonucléotide selon l'une des revendications 1-2 comme sonde d'acide nucléique ou comme amorce.

10. Trousse d'essai selon la revendication 9, caractérisée en ce que l'on effectue la recherche avec une sonde d'acide nucléique.

11. Trousse d'essai selon la revendication 9, caractérisée en ce que l'on effectue la recherche par amplification d'acide nucléique, dans laquelle on utilise comme amorce un oligonucléotide selon l'une des revendications 1-2.
